# EUROPÄISCHE PATENTANMELDUNG

(11) **EP 1 159 985 A1**
(43) Veröffentlichungstag der Anmeldung: **05.12.2001**
(21) Anmeldenummer: 00111776.1
(22) Anmeldetag: 03.06.2000
(51) Int. Cl.: A61N 1/36

(54) **Vorrichtung zur Suchtbefreiung von drogenabhängigen Patienten**

(71) Anmelder: HHS Handels AG, 8603 Schwerzenbach ZH (CH)
(72) Erfinder: Stühmer, Rolf, 21224 Rosengarten (DE)
(74) Vertreter: Troesch Scheidegger Werner AG

(57) **Zusammenfassung**

Ein Gerät sowie die Verwendung des Gerätes zur Suchtbefreiung drogenabhängiger Menschen basiert auf bestimmter transcutaner, elektrischer Nervenstimulation zur Aktivierung körpereigener Enkephaline.

## Beschreibung

Die vorliegende Erfindung betrifft eine Vorrichtung zur Suchtbefreiung drogenabhängiger Patienten sowie eine Verwendung der Vorrichtung für die Suchtbefreiung drogenabhängiger Personen.

Suchtkrankheiten, wie die Abhängigkeit von Drogen, Nikotin, Methadon, Alkohol und dgl. sind in der heutigen Gesellschaft sehr verbreitet und deren Bekämpfung bzw. die Heilung von diesen Krankheiten ist ein zentrales, gesellschaftspolitisches Anliegen.

Insbesondere die Suchtbefreiung von harten Drogen, wie Heroin, Kokain und dgl. ist sozialpolitisch ein wichtiger Aspekt, und Therapien die zur Suchtbefreiung führen können, werde deshalb höchste Aufmerksamkeit geschenkt.

An dieser Stelle soll nicht auf alle bekannten Therapien zur Suchtbefreiung eingegangen werden, da deren Anzahl enorm ist. Erfolgversprechende Therapien sind aber sehr selten und teilweise auch umstritten, da sie beispielsweise auf menschenunwürdigen Therapie-Methoden basieren, oder auf der kontrollierten Abgabe von Drogen, was beispielsweise von der Weltgesundheitsorganisation abgelehnt wird.

Es ist daher eine Aufgabe der vorliegenden Erfindung, eine Methode bzw. die Verwendung einer Vorrichtung vorzuschlagen, mittels welcher eine Suchtbefreiung drogenabhängiger Patienten unter menschenwürdigen Bedingungen ermöglicht wird.

Erfindungsgemäss wird die gestellte Aufgabe mittels einer Vorrichtung, gemäss dem Wortlaut nach Anspruch 1 sowie einer Verwendung der Vorrichtung, gemäss dem Wortlaut nach Anspruch 9, gelöst.

Das Gerät bzw. die Methode für die Suchtbefreiung drogenabhängiger Menschen basiert darauf, dass über zwei Elektroden, welche beidseits auf das Mastoid eines Patienten geklebt werden, elektrisch bipolare Pulse einstellbarer Amplitudengrösse, Pulsdauer und Frequenz appliziert werden, welche bewirken, dass Enkephaline an den Opiatrezeptoren freigesetzt werden.

Durch diese Art elektrischer Stimulation der Nervenzellen im Gehirn wird die Verfügbarkeit von Enkephaline an den Opiatrezeptoren erhöht, wodurch das von aussen zugeführte Opiat an den Rezeptoren verdrängt wird, das bisher die Rezeptoren blockierte und die Enkephalineproduktion unterdrückt.

Je nach dem zu verdrängenden Opiat wird eine andere, bestimmte Frequenz eingesetzt.

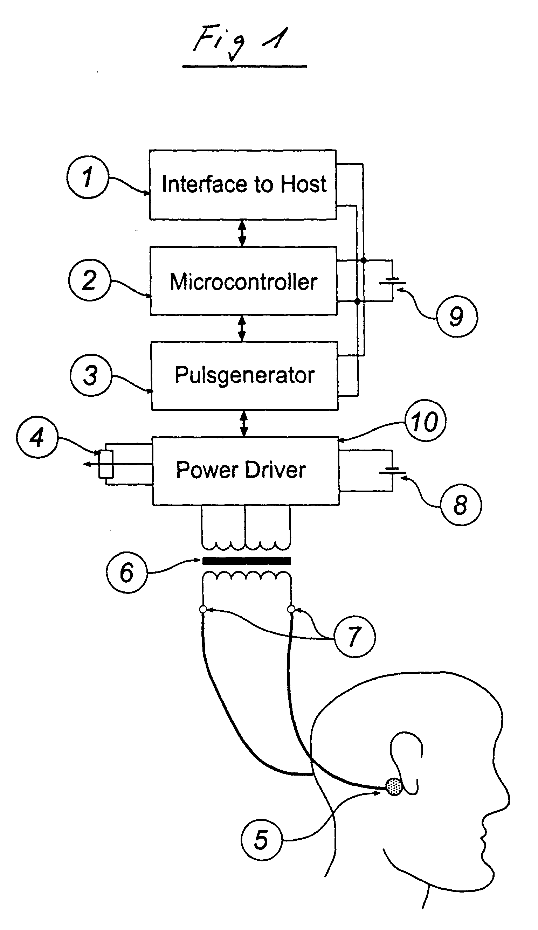

Für die Beschreibung der erfindungsgemässen Vorrichtung bzw. Verwendung der Vorrichtung für die Suchtbefreiung von Patienten wird auf die beigefügte Figur 1 verwiesen, in welcher die erfindungsgemässe Vorrichtung und die Uebertragung der Impulse auf den Patienten schematisch dargestellt ist.

Das Gerät wird mittels Interface 1 von einem Computer oder Hostcontroller auf die bestimmten Opiate programmiert, von denen der Mensch befreit werden soll. Der Mikrocontroller 2 übernimmt folgende Daten: Pulsdauer, Repetitions-Modulationsfrequenz, Applikationsdauer und Rhythmus. Nach erfolgter Programmierung wird die Kommunikation zwischen Gerät und Computer getrennt und mit zwei Elektroden 7 verbunden, welche am zu behandelnden Menschen beidseits auf das Mastoid 5 aufgeklebt werden.

Der Mikrocontroller 2 generiert dann über den Pulsgenerator 3 die bestimmten programmierten elektrischen Pulse und steuert den Power Driver 10, an dessen Ausgang ein Trenntransformator 6 für die Sicherheit des Menschen sorgt. Am Ausgang des Trenntransformators 6 werden die Elektroden angeschlossen und auf das Mastoid 5 geklebt. Die Reizspannung lässt sich über ein Bedienungselement 4 in einem vorgegebenen Bereich beliebig einstellen.

Gemäss einer Ausführungsvariante der vorliegenden Erfindung wird der Power Driver 10 von einer separaten Stromquelle 8 versorgt, die sich während längeren Behandlungszeiten beliebig austauschen lässt, ohne dass die Programmierdaten verloren gehen, welche über eine Stützbatterie gesichert bleiben.

Beim Interface 1 kann es sich um eine optische, induktive, elektromagnetische oder elektrische Kopplung mit dem Computer handeln.

Dem Power Driver 10 können beliebige Frequenzmuster aufgeprägt werden, sei es aus extern anschliessbaren Quellen oder aus im Gerät selbst erzeugten, aufrufbaren Mustern (z.B. "weisses" oder "rosa" Rauschen, Musik, Text, etc.).

Die Haftelektroden können zusätzlich mit Schallgebern versehen sein, die den hörbaren Bereich und den Ultraschallbereich abdecken. Bei den Schallgebern kann es sich um Piezowandler handeln.

Vorzugsweise können der Betriebszustand und die Betriebsdaten sowie weitere spezifische Daten durch Fernabfrage festgestellt werden.

Das Programmiergerät kann ein Computer, ein Hostcontroller oder eine am Gerät selbst angebrachte Programmiereinheit sein.

Gemäss einer weiteren Ausführungsvariante erfolgt die Einstellung der Reizspannung nicht durch das Bedienungselement 4, sondern wird automatisch über den Hautwiderstand nachgeführt, der an den Elektroden gemessen wird. Die Pulsdauer und Repetitionsfrequenz lassen sich automatisch oder manuell durch ein sogenanntes Feedback nachführen bzw. korrigieren, wobei das Feedback durch Messung des Hautwiderstandes erfolgt, welcher unter Umständen über den Erregungszustand des Patienten Aufschluss gibt.

Für das Gerät und die damit zu behandelnden Süchte werden folgende Festfrequenzen verwendet:
Nikotin: ca. 5 - 10 Hz;
Ketamine (Methadon): ca. 25 - 35 Hz, vorzugsweise ca. 30 Hz;
Heroin: ca. 65 - 85 Hz, vorzugsweise 70 - 80 Hz;
Barbiturate, Alkohol: 400 - 600 Hz, vorzugsweise ca. 500 Hz;
Kokain: ca. 1800 - 2200 Hz, vorzugsweise ca. 2000 Hz.

Als Pulsform wird ein bipolarer Rechteckpuls verwendet bei einer Pulsdauer von ca. 250µs/Periode. Die Reizspannung ist am Potentiometer einstellbar von 30V - 100V.

Bei den obigen Angaben handelt es sich selbstverständlich um Beispielsangaben, die für das bessere Verständnis für die vorliegende Erfindung dienen sollen. Schlussendlich sind die einstellbaren Festfrequenzen Patienten-abhängig, werden während der Behandlungsdauer und abhängig vom Fortschritt verändert und sind entsprechend so anzupassen, dass sie beim jeweiligen Patienten zur gewünschten Wirkung bzw. Suchtbefreiung führen.

## Patentansprüche

1. Vorrichtung zur Suchtbefreiung drogenabhängiger Patienten, **gekennzeichnet durch**
- zwei Elektroden (7), vorgesehen um beidseits auf das Mastoid (5) des Patienten geklebt zu werden,
- einen Mikrocontroller (2), welcher mittels Interface (1) von einem Computer oder dgl. auf bestimmte Optiate programmierbar ist, sowie
- einen Pulsgenerator (3), über welchen **durch** den Mikrocontroller bestimmte, programmierte, elektrische Pulse generierbar sind, welche über die Elektroden appliziert werden.

2. Vorrichtung nach Anspruch 1, **dadurch gekennzeichnet, dass** ein sogenannter Power Driver (10) nachgeschaltet zum Pulsgenerator (3) vorgesehen ist und von diesem angesteuert wird, sowie ein Trenntransformator (6) an dessen Ausgang die Elektroden (7) angeschlossen sind.

3. Vorrichtung nach Anspruch 2, **dadurch gekennzeichnet, dass** der Power Driver (10) von einer separaten Stromquelle (8) versorgt wird, welche austauschbar ist, ohne dass die im Power Driver vorhandenen Programmierdaten verloren gehen, indem eine zusätzliche Stützbatterie (9) vorgesehen ist.

4. Vorrichtung nach einem der Ansprüche 1 bis 3, **dadurch gekennzeichnet, dass** es sich beim Interface (1) um eine induktive, elektromagnetische oder elektrische Koppelung mit dem Computer handelt.

5. Vorrichtung nach einem der Ansprüche 2 bis 4, **dadurch gekennzeichnet, dass** am Power Driver (10) ein externes Gerät anschliessbar ist zum Uebertragen bzw. Aufprägen eines beliebigen Frequenzmusters.

6. Vorrichtung nach einem der Ansprüche 1 bis 5, **dadurch gekennzeichnet, dass** es sich bei den Elektroden um Haftelektroden handelt, welche mit zusätzlichen Schallgebern, wie beispielsweise Piezowandlern versehen sind, welche den hörbaren sowie Ultraschallbereich abdecken.

7. Vorrichtung nach einem der Ansprüche 1 bis 6, **dadurch gekennzeichnet, dass** eine Fernabfrage vorgesehen ist für die Feststellung des Betriebszustandes, der Betriebsdaten sowie weiterer Geräte-spezifischer Daten.

8. Vorrichtung nach einem der Ansprüche 1 bis 7, **dadurch gekennzeichnet, dass** mindestens ein Messorgan vorgesehen ist, zur Messung des Hautwiderstandes, welcher Aufschluss über den Erregungszustand eines Patienten geben kann, um gegebenenfalls Pulsdauer und Repetitionsfrequenz nachzuführen bzw. zu korrigieren.

9. Verwendung einer Vorrichtung für die Suchtbefreiung drogenabhängiger Personen, **dadurch gekennzeichnet, dass** zwei Elektroden vorgesehen sind, um beidseits auf das Mastoid eines Patienten geklebt zu werden, und dass elektrisch bipolare Pulse einstellbarer Amplitudengrösse, Pulsdauer und Frequenz erzeugbar und applizierbar sind, welche bewirken, dass Enkephaline an den Opiatrezeptoren freisetzbar sind.

10. Verwendung nach Anspruch 9, **dadurch gekennzeichnet, dass** es sich um eine Vorrichtung nach einem der Ansprüche 1 bis 8 handelt.

11. Verwendung nach einem der Ansprüche 9 oder 10, **dadurch gekennzeichnet, dass** für die zu behandelnden Süchte die folgenden Festfrequenzen verwendet werden:
Nikotin: ca. 5 - 10 Hz;
Ketamine (Methadon): ca. 25 - 35 Hz, vorzugsweise ca. 30 Hz;
Heroin: ca. 65 - 85 Hz, vorzugsweise 70 - 80 Hz;
Barbiturate, Alkohol: 400 - 600 Hz, vorzugsweise ca. 500 Hz;
Kokain: ca. 1800 - 2200 Hz, vorzugsweise ca. 2000 Hz.

12. Verwendung nach einem der Ansprüche 9 bis 11, **dadurch gekennzeichnet, dass** es sich bei der Pulsform um einen bipolaren Rechteckpuls handelt, sowie eine Pulsdauer gewählt wird von ca. 250 - 300µs/Periode, vorzugsweise 250µs/Periode, und dass am Potentiometer eine Reizspannung einstellbar ist im Bereich von ca. 30V - ca. 100V.
